# EUROPEAN PATENT APPLICATION

(11) **EP 4 585 899 A1**
(43) Date of publication of application: **16.07.2025**
(21) Application number: 23863211.1
(22) Date of filing: 06.09.2023
(51) Int. Cl.: G01N 1/04, G01N 1/28

(54) **STOOL COLLECTION SYSTEM**

(30) Priority: 07.09.2022 JP 2022142442
(71) Applicant: University Public Corporation Osaka, Osaka-shi, Osaka 5360025 (JP); Iida Sangyo Co., Ltd., Musashino-shi, Tokyo 180-0022 (JP)
(72) Inventor: FUNANO Shunichi, Osaka-shi, Osaka 558-8585 (JP); MORI Kazuhiko, Musashino-shi, Tokyo 180-0022 (JP); MATSUMOTO Koichi, Musashino-shi, Tokyo 180-0022 (JP)
(74) Representative: Mewburn Ellis LLP
(86) International application number: PCT/JP2023/032531
(87) International publication number: WO 2024/053673

(57) **Abstract**

The present invention provides a stool collection system that can automatically collect and prepare the entirety of stool from a test subject on an ongoing basis. The stool collection system according to the present invention is characterized by comprising: a preparation chamber that is communicated with a toilet seat; a conveyance mechanism that disposes a container for collecting the stool at a prescribed position with respect to the toilet seat, and that recovers, into the interior of the preparation chamber, the container in which the stool was loaded; a drier for drying the recovered stool; a crusher that crushes the dried stool; and a packaging machine for packaging the crushed stool.

## Description

The present invention relates to a collection and preparation techniques of stool sample.

### BACKGROUND ART

Various bacteria that live in the human intestine form the intestinal flora, which is said to number more than 1,000 species and approximately 100 trillion. In particular, the large intestine is home to a wide variety of intestinal bacteria, and the state of the intestinal flora differs from person to person and is thought to change depending on lifestyle, age, stress, and the like.

By examining this intestinal flora, it is possible to find associations with a variety of diseases, including intestinal diseases (colon cancer, ulcerative colitis, and the like.), as well as liver cancer, diabetes, allergies, depression, rheumatism, and dementia. Therefore, it is expected that in the future, testing of the intestinal flora will be used to diagnose health and disease. Furthermore, by understanding the composition of intestinal bacteria, the information can be used to provide advice on dieting, improving eating habits, and alleviating skin problems, and the like, and can be useful in daily of life.

In the conventional intestinal bacteria tests, the subjects themselves collect a portion of their stool after defecation and send it to a testing laboratory, where the intestinal flora is analyzed and the results are communicated to the subjects at a later date. While most of the analysis steps have been automated and optimized, the stool collection step is manual, and each collection is a burden for the subject.

Many subjects collect stool samples at home by using a test kit (see, for example, Patent Document 1). However, sampling is time-consuming depending on constipation or the timing of defecation, and the complicated sampling process makes it difficult to perform tests over time. Further, because the samples are so small, there is a possibility that the test results will be biased.

### PRIOR ART REFERENCE

### Non-Patent Reference

Patent Document 1: JP 2021-51001 A

### SUMMARY OF THE INVENTION

### Problem to be solved by the invention

In view of the above-mentioned conventional techniques, an object of the present invention is to provide a stool collection system that can automatically collect and prepare the entirety of stool from a test subject on an ongoing basis.

### MEANS TO SOLVE THE PROBLEMS

In order to solve the above-mentioned problems, the present invention provides a stool collection system characterized by comprising:
a toilet bowl having a toilet seat;
a conveyance mechanism for placing a stool collection member at a predetermined position below the toilet seat, and conveying the stool collection member on which the stool is loaded out of the toilet bowl;
a preparation chamber for recovering the conveyed stool; and
a packaging machine for packaging the recovered stool.

In the description, the toilet seat is used as a concept including a toilet bowl. Further, the stool collection system may also be referred to as a stool collection device. Therefore, after the stool is collected and recovered, processes such as deoxidation, refrigeration, freezing, drying, crushing, and analysis may be carried out in the preparation chamber, or the recovered and packaged stool may be delivered to an external institution to be analyzed by the institution.

In the stool collection system of the present invention, it is preferable that the stool collection member includes a water-soluble sheet and/or a container.

Further, it is preferable that the stool collection system of the present invention further comprises a dryer for drying the recovered stool, and a crusher for crushing the dried stool.

It is preferable that the stool collection system of the present invention further comprises a molding machine for molding the recovered stool before drying.

Further, in the stool collection system of the present invention, it is preferable that the molding machine is a press machine that presses the stool loaded in the container from above. It is preferred that the press machine presses the stool through a film.

Further, in the stool collection system of the present invention, it is preferable that the packaging machine divides the crushed stool into small portions and packages them.

### EFFECT OF THE INVENTION

According to the present invention, the entire amount of the stool from a subject can be collected automatically and continuously. Thus, the state of the stool can be easily preserved and subsequent stool analysis can be easily performed. Furthermore, by collecting the entire amount of the stool, the characteristics of the stool can be understood without exception. Therefore, it is possible to analyze the intestinal flora over time, which can clarify the relationship with disease and contribute to health promotion. This will contribute to accelerating life science research and improving the health of subjects.

### BRIEF EXPLANATION OF DRWAINGS

FIG. 1 is a schematic diagram of the stool collection system 10 according to the embodiment 1.
FIG. 2 is a schematic diagram of the process from collection to preparation of the stool 3 in the embodiment 1.
FIG. 3 is a schematic diagram showing an example of the molding process of the stool 3.
FIG. 4 is a schematic diagram showing another example of the molding process of the stool 3.
FIG. 5 is a schematic diagram showing another example of the molding process of the stool 3.
FIG. 6 is a schematic diagram showing yet another example of the molding process of the stool 3.
FIG. 7 is a flowchart showing the process from collection to preparation of the stool 3.
FIG. 8 is a schematic diagram of the stool collection system 10 according to the embodiment 2.
FIG. 9 is a schematic diagram of the process from collection to preparation of the stool 3 in the embodiment 2.

### MODE FOR CARRYING OUT THE INVENTION

In the following, by referring the drawings, the typical embodiments of the stool collection system according to the present invention are explained in detail. However, the present invention is not limited to the drawings. Further, since these drawings are presented to explain the concept of the present invention, there are cases where sizes, ratios and numbers are exaggerated or simplified as necessary for ease of understanding.

### 1. Embodiment 1

### 1-1. Configuration of the stool collection system 10

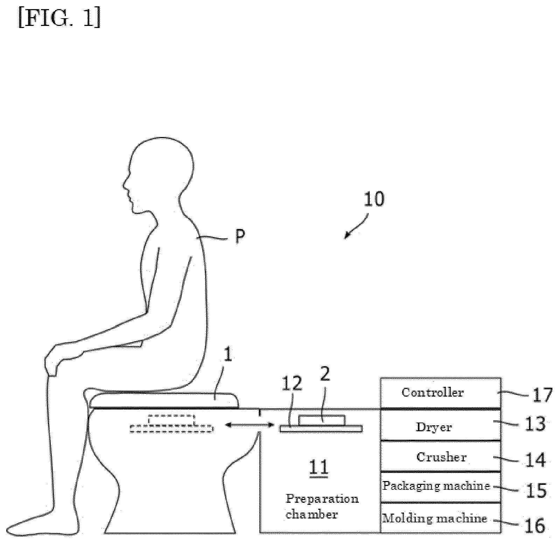

A stool collection system 10 according to embodiment 1 will be described with reference to FIG. 1 to FIG. 7. The stool collection system 10 may also be referred to as a stool collection device.

The stool collection system 10 includes a preparation chamber 11, a conveyance mechanism 12, a dryer 13, a crusher 14, and a packaging machine 15, and is configured integrally with a toilet seat 1 or attached to the toilet seat 1 or the toilet bowl, and automatically collects a stool 3 from a subject P. A typical subject P is assumed to be a resident of a house in which the toilet seat 1 is installed, but is not limited thereto. For example, the subject P may be a user of other facilities such as hospitals, health checkup centers, and nursing homes. In addition, the toilet seat 1 in this embodiment includes a toilet bowl.

As shown in FIG. 1, the stool collection system 10 can be configured to include the preparation chamber 11, the conveyance mechanism 12, the dryer 13, the crusher 14, the packaging machine 15, and a controller 17.

The stool collection system 10 utilizes a container 2, which is a stool collection member, to collect the stool 3. The container 2 may or may not be included as a component of the stool collection system 10. The container 2 is, for example, a sheet, tray or dish on which the stool 3 is loaded, and preferably has a peripheral wall, but does not necessarily have a peripheral wall. The container 2 is made of, for example, paper or a resin such as polyvinyl alcohol (PVA), is water-soluble and/or flammable, and is disposable.

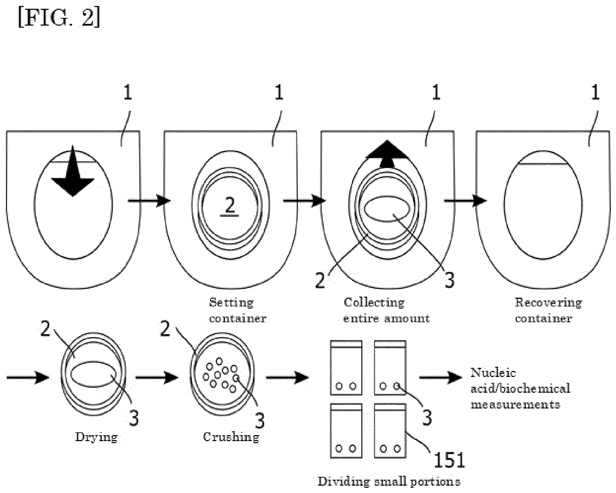

Note that, in the example of FIG. 2, although the entire amount of the stool 3 is collected, from the viewpoint of preserving an appropriate amount, a portion may be collected and the remainder may be discarded. In the former case, the container 2 having a capacity suitable for collecting the entire amount is used. In the latter case, in order to prevent bias in the collected stool, it is preferable to knead (mix) the stool 3 in the container 2 with a kneading tool (not shown) and collect the kneaded stool 3. As the kneading tool, for example, it is suitable to use a pair of rollers that knead the stool 3 by kneading the stool 3 from both sides of the peripheral wall of the container 2. In response to such rollers, it is suitable to use a relatively flexible container 2 that can be deformed by pressure from the circumferential direction. Instead of the rollers, a balloon or the like used in a massage machine may be used.

The preparation chamber 11 communicates with the toilet seat 1 to allow movement of the container 2 between the toilet seat 1 and the preparation chamber. As will be described in detail later, in this embodiment, the conveyed stool 3 is prepared, that is, dried, crushed and packaged, in the preparation chamber 11, but the preparation may also be performed in a different location. In the latter case, the preparation chamber may be referred to as a temporary storage chamber for the stool 3.

Although one preparation chamber 11 is illustrated in FIG. 1, the preparation chamber 11 may be provided for each preparation process. For example, a preparation chamber for drying (drying chamber), a preparation chamber for crushing (crashing chamber), and a preparation chamber for packaging (packaging chamber) may be provided. Furthermore, each preparation chamber may be sealed off from the outside.

The conveyance mechanism 12 places the container 2 at a predetermined position below the toilet seat 1, and conveying the container 2 on which the stool 3 is loaded to the preparation chamber 11. An example of the conveyance mechanism 12 is a rack that conveys the container 2 from the toilet seat 1 to the preparation chamber 11 or from the preparation chamber 11 to the toilet seat 1 by a motor (not shown). Alternatively, as another example, the conveyance mechanism 12 may be an arm (not shown) that moves the container 2.

The conveyance mechanism 12 starts operating when the subject operates an operation panel (not shown) of the controller 17, for example, by selecting the subject and pressing a start button. For example, the conveyance mechanism 12 loads the container 2 in the preparation chamber 11 and sends the container 2 to a predetermined position on the toilet seat 1.

The conveyance may be performed automatically after a sensor (for example, a weight sensor) detects that stool excretion has ended, or the subject may turn on a conveyance switch when stool excretion has ended.

Note that, when partial collection of the stool 3 is performed, the stool 3 is kneaded with a kneading tool and the stool 3 is partially discarded before or after the conveyance.

Furthermore, in the case where the preparation chamber 11 is provided for each preparation process, the conveyance mechanism 12 may include a belt conveyor that comes and goes between a plural of the preparation chambers.

The dryer 13 dries the recovered stool 3 within the preparation chamber 11. From the viewpoints of suppressing the effect of drying on the nucleic acids of the intestinal bacteria in the stool 3, of uniformly drying the stool 3, and of easiness of handling after drying, lyophilization (so-called freeze-drying) is preferably utilized as the drying method, but is not limited thereto, and for example, microwave heating drying, simple heating drying, and the like may also be used. Alternatively, quick freezing using liquid nitrogen or freezing in a freezer may also be utilized. When using a freezer, from the viewpoint of preserving the composition of the intestinal flora, it is desirable that the freezer has the performance to freeze the stool 3 to a predetermined temperature (for example, approximately -18°C) within a predetermined time (for example, within 15 minutes).

Here, the freeze-drying is a method of removing water from a frozen sample by sublimating ice through reduced pressure, and may be carried out under the pressure conditions that allow the water to change from a solid to a gaseous state in the phase diagram, but is preferably carried out under conditions of 100 Pa or less. More preferably, the freeze-drying is carried out under conditions of 50 Pa or less.

The standard drying time is, for example, 24 hours, but since the drying time varies depending on the moisture content of the stool 3, the drying time may be adjusted depending on the condition of the stool 3 (including the moisture content). For example, the measured weight of the stool 3 or the weight change per unit time may be used as an indicator of the state of the stool 3, and drying may be terminated when the weight becomes lighter than a set value or when the weight change per unit time becomes smaller than a set value.

The dryer 13 as a freeze-dryer may be configured to include, for example, a main body (trap portion) that traps the sublimated water, a dry chamber in which the sample is loaded, a pressure reducing device (vacuum pump) that reduces the pressure inside the system to promote sublimation, and a preliminary freezing device (preliminary freezing tank, freezer) that pre-freezes the container containing the sample (none of the components are shown).

The crusher 14 crushes the dried stool 3 so that it is finely and uniformly dispersed (see FIG. 2). The crusher 14 may be capable of crushing the stool 3 into a size having a diameter of approximately 0.01 mm to 5 mm. When the particle size after the crushing is too small, the nucleic acids in the stool 3 are broken down and cannot be analyzed, whereas when the particle size after the crushing is too large, the stool 3 is not available to handle.

The crusher 14 is assumed to be, for example, a mechanical crusher, but may be a sonic crusher or the like. The mechanical crusher 14 crushes the stool 3 by compressing or impacting the stool 3 from above with, for example, a hammer or roller (not shown).

The packaging machine 15 packages the crushed stool 3 (see FIG. 2). The packaging machine 15 can divide and package the crushed stool 3 into small portions, for example, dividing the crushed stool 3 into moisture-proof and water-resistant containers or bags 151 at fixed weight portions. For example, the stool may be divided into small portions and packaged in a series of blister packs, and in this case, for example, the name of the subject and collection date and time may be written or printed on the pack by using information registered in the controller 17.

The stool collection system 10 may be configured such that the recovered stool 3 is molded in the molding machine 16 before being dried to increase its surface area. Since moisture evaporates from the surface of the stool 3, increasing the surface area of the stool 3 is advantageous for drying the stool 3. Moreover, by molding the surface area of the stool 3 to be large, the drying time can be shortened and deterioration of the stool 3 can be suppressed. For example, a thin, approximately plate-like shape is preferable, and further, the surface of the stool 3 may be made uneven to increase the surface area.

From the viewpoint of suppressing the occurrence of parts that need to be cleaned, the molding machine 16 may have the following configuration.

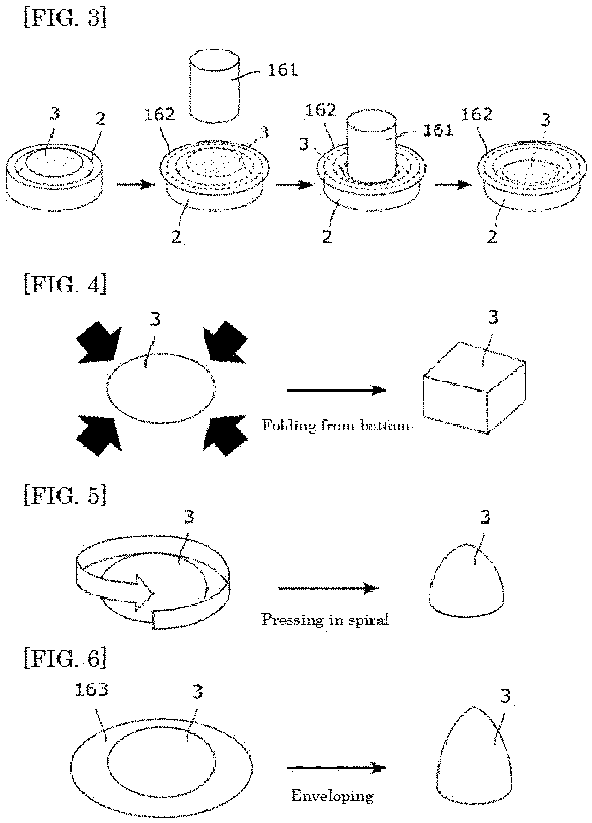

That is, it is preferable that the molding machine 16 is a press machine that presses the stool 3 in the container 2 from above. In this case, the molding machine 16 kneads the stool 3 inside the container 2 by smushing it from above (see FIG. 3). A club or a disc with an uneven surface may also be used.

The area and shape of the working portion 161 (pressing portion or slide) of the molding machine 16 may be set appropriately depending on the container 2. In order to avoid the need to perform a cleaning process for the working portion 161, a cover 162 may be attached to the working portion 161.

It is preferable that the cover 162 is disposable and is attached to the working portion 161 before molding and becomes integral with the container 2 after molding. The material of the cover 162 is not particularly limited, but from the viewpoint of disposal, it is preferable that the material is the same as that of the container 2.

When the area of the working portion 161 of the molding machine 16 is small, it is preferable that the cover 162 has a size that can be accommodated inside the container 2 so that the stool 3 can be reliably smushed.

On the other hand, when the area of the pressing surface of the working portion 161 corresponds to the container 2, the shape of the cover 162 is not important, and it may be a film that protects the working portion 161. At that time, the container 2 may be covered with a film before molding.

As another example, the molding machine 16 may be one that encapsulates the stool 3.

That is, the molding machine 16 deforms the flexible container 2 so as to encase the stool 3 in the container 2, and kneads the stool 3 inside by smushingt (see FIG. 4).

For example, when the molding machine 16 encases the stool 3 together with the dish-shaped container 2, a force is applied from the bottom side of the container 2 so as to fold and encase (see FIG. 5).

Alternatively, a film 163 for enveloping and preventing leakage may be provided on the outside of the container 2, and the stool 3 may be enveloped together with the film 163 (see FIG. 6). In this case, the force is not limited to being applied from below, but may be applied from the side. The material of the film 163 is preferably the same as the material of the container 2.

Alternatively, a mold (not shown) may be provided to apply force from above.

The controller 17 controls the conveyance mechanism 12, the dryer 13, the crusher 14, the packaging machine 15 and the molding machine 16. For example, the controller 17 controls the conveyance mechanism 12 to convey the empty container 2 to the toilet bowl 1 and to recover the container 2 on which the stool 3 is loaded into the preparation chamber 11 in response to the operation of the subject P. The controller 17 also controls the molding machine 16 to mold the recovered stool 3 into a desired size and shape. Next, the controller 17 controls the dryer 13 to dry the molded stool 3 under predetermined conditions. Thereafter, the controller 17 controls the crusher 14 to crush the dried stool 3 to a desired size. The controller 17 controls the packaging machine 15 to divide the crushed stool 3 into portions of desired weight and package.

In addition, the stool collection system 10 may have a supply mechanism that supplies the container 2 to the conveyance mechanism 12, and a placement mechanism that places the container 2 on which the stool 3 is loaded at a predetermined location within the preparation chamber 11, neither of which are shown in the figure.

### 1-2. Working operation of the stool collection system 10

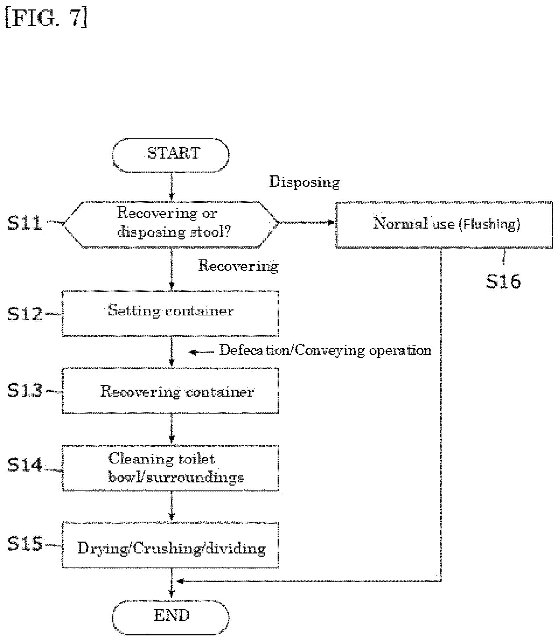

Next, the working operation of the stool collection system 10 will be explained with reference to FIG. 7.

The stool collection system 10 accepts a selection of whether to recover or discard the stool 3 (step S11). When the subject P selects to discard the stool 3, the stool collection system 10 goes into normal use mode (step S16), the stool 3 is flushed away by the toilet bowl 1, and the series of procedures is completed.

On the other hand, when the subject P selects to recover the stool 3, the stool collection system 10 goes into recovery mode and the following procedures are carried out.

In the recovery mode, before the subject P defecates, the container 2 is conveyed to the toilet seat 1 and set in a predetermined position (step S12). After defecation, the container 2 is carried over the wall of the toilet seat 1 to the adjacent preparation chamber 11 in response to an operation by the subject P or automatically in response to the measurement result (detection) of the weight sensor (step S13). At the same time, the toilet bowl 1 and the like are flushed (step S14). The stool 3 brought into the preparation chamber 11 is dried, crushed, and packaged (step S15). Molding may be carried out prior to the drying. Then, the series of procedures is completed.

The stool collection system 10 repeats the above procedures. These procedures may be performed each time the subject P defecates, or may be performed for each of multiple subjects P, for example, once a day.

The packaged stool 3 may be stored under frozen or refrigerated conditions, or at room temperature, so long as the dried condition is kept. It is desirable that the stool 3 is stored while maintaining the composition of the intestinal flora at the time of collection. For example, it is preferable that the Spearman's rank correlation coefficient of the intestinal flora composition is maintained at 0.8 or higher. Further, it is preferable that an index for measuring the degree of similarity, such as the Jaccard distance, the Bray-Curtis distance, or the Unifrac distance, is close to 0 (for example, 0.1 or less).

The packaged stool 3 is subjected to nucleic acid and biochemical measurements (intestinal flora analysis, fecal occult blood test, and the like).

### 1-3. Effects of embodiment 1

According to the embodiment 1, the entire amount of the stool 3 can be collected automatically, and therefore the characteristics of the stool 3 can be grasped without omission. Further, since the stool 3 is collected continuously and the composition of the intestinal flora in the stool 3 collected in the past can be retained for a long period of time, it is possible to perform an intestinal flora analysis over time and, then, to understand the health of the subject P.

Further, since the state of the stool 3 can be easily maintained, analysis of the stool 3 is easy. The stored stool 3 can also be analyzed by using new techniques that were not available at the time of collection. Therefore, by clarifying the relationship with disease, it contributes to improving the health of subject P and also contributes to accelerating life science research.

### 2. Embodiment 2

### 2-1. Configuration of the stool collection system 20

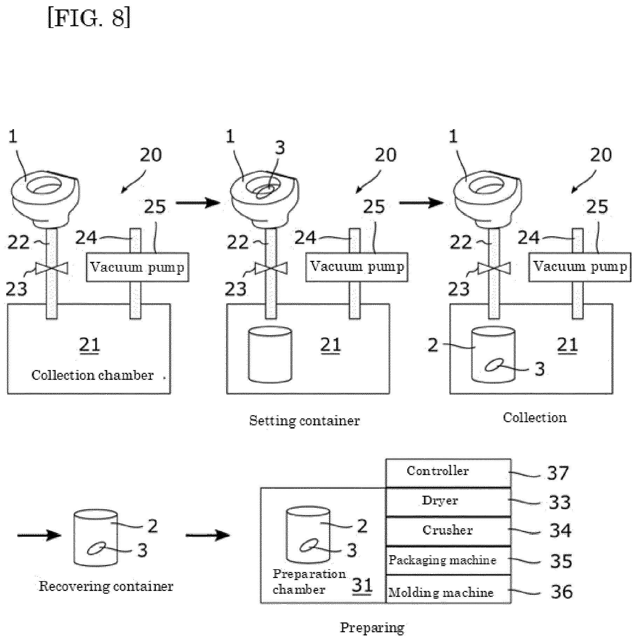

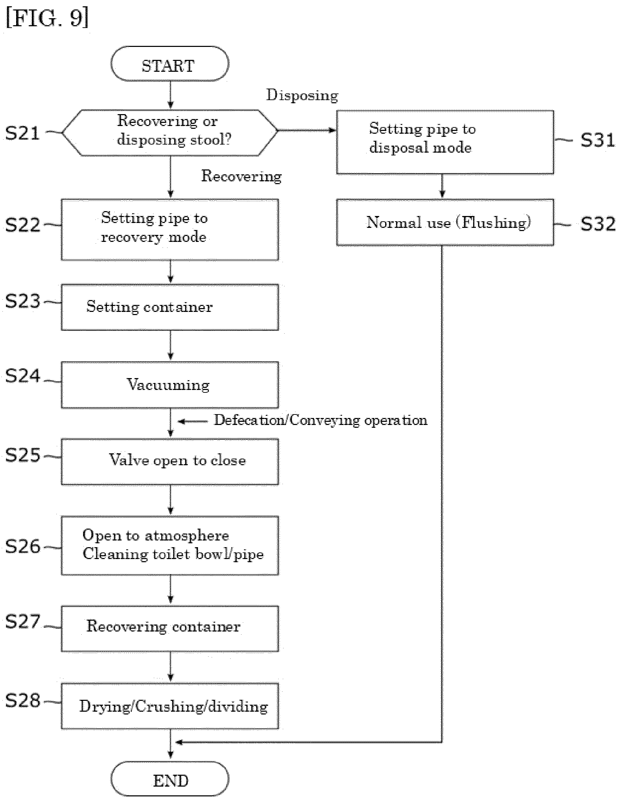

The stool collection system 20 according to the embodiment 2 will be described with reference to FIG. 8 and FIG. 9.

In the embodiment 2, the stool 3 is conveyed to a collection chamber 21 outside the toilet room, where the stool 3 is collected (see FIG. 8). Therefore, the stool collection system 20 includes a collection chamber 21, a preparation chamber 31, a dryer 33, a crusher 34, a packaging machine 35, and a molding machine 36.

Here, the preparation chamber 31, the dryer 33, the crusher 34, the packaging machine 35, and the molding machine 36 can be used as the same components as the preparation chamber 11, the dryer 13, the crusher 14, the packaging machine 15, and the molding machine 16 in the embodiment 1. Therefore, the following description will focus on the collection chamber 21.

The collection chamber 21 accommodates the container 2 for collecting the stool 3. The collection chamber 21 includes a pipe 22 that connects to the toilet seat 1 and a pipe 24 that connects to a vacuum pump 25.

The pipe 22 has a valve 23 that opens and closes the pipe 22. The vacuum pump 25 sucks the gas in the collection chamber 21 to make the pressure within the sampling chamber 21 to a negative pressure relative to the toilet seat 1. The working operation of the valve 23 and vacuum pump 25 will be described in detail later.

The stool collection system 20 is controlled by a controller 37. That is, the controller 37 controls the operation of the valve 23 and the vacuum pump 25 , and also controls the dryer 33 , the crusher 34 , the packaging machine 35 , and the molding machine 36.

### 2-2. Working operation of the stool collection system 20

The working operation of the stool collection system 20 will be explained with reference to FIG. 9.

The stool collection system 20 accepts a selection of whether to recover or discard the stool 3 (step S21). When the subject P selects to discard the stool 3, the stool collection system 20 goes into normal use mode. That is, the pipe 22 is set to the exhaust mode (step S31), and the stool 3 is flushed away by the toilet bowl 1 (step S32), and the series of procedures is completed.

On the other hand, when the subject P selects to recover the stool 3, the stool collection system 10 goes into recovery mode and the following procedures are carried out.

The stool collection system 10 sets the pipe 22 to a recovery mode (i.e., closes the valve 23; step S22) before defecation, and places the container 2 in the collection chamber 21 (step S23). Then, the vacuum pump 25 is operated to reduce the pressure inside the collection chamber 21 (step S24).

After defecation, the valve 23 opens in response to the operation of the subject P (step S25), and the stool 3 is sucked into the collection chamber 21 due to the air pressure difference between the inside of the toilet room and the inside of the collection chamber 21. The stool 3 that reaches the collection chamber 21 is accommodated in the container 2.

Thereafter, the stool collection system 20 closes the valve 23, and then opens the inside of the collection chamber 21 to the atmosphere (step S26). At the same time, the toilet bowl 1 and the pipes 22 are washed.

The container 2 is taken out from the collection chamber 21 and conveyed to the preparation chamber 31 (step S27). The conveyed stool 3 is dried by the dryer 33, crushed by the crusher 34, and packaged by the packaging machine 35 (step S28). The stool 3 brought into the preparation chamber 31 may be molded by the molding machine 36 before drying.

Then, the series of procedures is completed. The stool collection system 20 repeats the above procedures.

### 2-3. Effects of the embodiment 2

According to the embodiment 2, the entire amount of the stool 3 can be collected automatically, and therefore the characteristics of the stool 3 can be grasped without omission. Moreover, since the stool 3 is collected continuously, it is possible to perform the intestinal flora analysis over time.

The stool collection system 20 can also be used in areas and settings where water resources are at a premium. For example, it is possible to use in aircraft, ships, and the like.

Further, since the state of the stool 3 can be easily maintained, analysis of the stool 3 can be easily carried out. Therefore, by clarifying the relationship with disease, it contributes to improving the health of subject P and also contributes to accelerating life science research.

Representative embodiments of the present invention have been described above, but the present invention is not limited thereto, and various design modifications are possible, which are also included in the present invention. For example, a dryer/crusher in which a dryer and a crusher are integrated may be used.

In the above embodiments, examples have been described where a tray or dish-shaped container 2 is used as the stool collection member, but a disposable sheet made of, for example, paper or a resin such as polyvinyl alcohol (PVA) and which is water-soluble and/or flammable may also be used. The sheet may have a double structure, and may have a folded bellows-like shape and a structure that can extend downward due to the weight of the stool to form a storage space. Also, a sheet may be used together with the container 2.

Further, in the above embodiment, the example has been described where the recovered stool 3 is prepared, that is, dried, crushed and packaged, in the preparation chamber 11 adjacent to the toilet bowl, but the recovered stool 3 may be only packaged in the preparation chamber 11 and delivered to an external institution or the like, where the stool is dried, crushed and analyzed.

Although the above embodiment employs collection of the entire amount of the stool 3, collection of only a portion of the stool 3 may be employed. In this case, the collected stool 3 can be individually packaged in containers such as blister packs and the individually packaged stool 3 can be frozen, eliminating the need for the crusher and the molding machine. An oxygen scavenger such as silica gel may be contained in the container.

### INDUSTRIAL APPLICABILITY

According to the stool collection system 10 of the present invention, the process from the stool collection to analysis can be completed within the home or a single facility. This is extremely beneficial for managing the health of the subject P. For example, the analysis results can be collected as data on an external server and made available, with the permission of the subject, to doctors and other medical and nursing professionals, which is expected to improve the health of the subject P.

### EXPLANATION OF SYMBOLS

1 Toilet seat
2 Container
3 Stool
10 Stool collection system
11 Preparation chamber
12 Conveyance mechanism
13 Dryer
14 Crusher
15 Packaging machine
16 Molding machine

## Claims

1. A stool collection system **characterized by** comprising:
a toilet bowl having a toilet seat;
a conveyance mechanism for placing a stool collection member at a predetermined position below the toilet seat, and conveying the stool collection member on which the stool is loaded out of the toilet bowl;
a preparation chamber for recovering the conveyed stool; and
a packaging machine for packaging the recovered stool.

2. The stool collection system according to claim 1, wherein the stool collection member includes a water-soluble sheet.

3. The stool collection system according to claim 1 , wherein the stool collection member includes a container.

4. The stool collection system **characterized by** further comprising a dryer for drying the collected stool, and a crusher for crushing the dried stool.

5. The stool collection system according to claim 4, further comprising a molding machine for molding the collected stool before drying.

6. The stool collection system according to claim 5, wherein the molding machine is a press machine that presses the stool loaded in the container from above.

7. The stool collection system according to claim 6, wherein the press machine presses the stool through a film.

8. The stool collection system according to claim 4, wherein the packaging machine divides the crushed stool into small portions and packages them.
